# EUROPEAN PATENT APPLICATION

(11) **EP 4 053 528 A1**
(43) Date of publication of application: **07.09.2022**
(21) Application number: 21160900.3
(22) Date of filing: 05.03.2021
(51) Int. Cl.: G01L 1/20, G01L 1/22, A43B 13/12, A43B 3/00, A61B 5/00

(54) **SENSOR ARRANGEMENT FOR DETECTING A FORCE BETWEEN A FOOT AND A SUPPORTING SURFACE**

(71) Applicant: MCG Motion Capture GmbH, 69123 Heidelberg (DE); Senmotion GmbH, 12435 Berlin (DE)
(72) Inventor: VOSS, Hartmut, 14473 Potsdam (DE); ERTELT, Thomas, 12557 Berlin (DE); KWIATEK, Andre, 12159 Berlin (DE)
(74) Representative: Maiwald Patent- und Rechtsanwaltsgesellschaft mbH

(57) **Abstract**

This disclosure provides a sensor arrangement (100) for detecting a force between a foot and a supporting surface. The sensor arrangement has an improved durability while allowing an accurate measurement of the force. The sensor arrangement (100) comprises:
a number of foil-based sensor elements (120), arranged as individual elements in an at least substantially common plane formed by a carrier (110);
a number of pad elements (130), the number of which corresponds to the number of sensor elements (120), and which are arranged as individual elements in a manner that an area extension of a respective one of the number of pad elements (130) at least partly overlaps with an area extension of a corresponding one of the number of sensor elements (120).

## Description

### FIELD OF THE INVENTION

The present invention relates to the field of sports medicine and/or gait analysis, and in particular to a sensor arrangement for detecting a force between a foot and a supporting surface. Further, the invention relates to a sole arrangement for a shoe, a detection system for detecting a force between a foot and a supporting surface, and a method for providing a sensor arrangement.

### BACKGROUND OF THE INVENTION

For example, in sports medicine, gait analysis, etc., there are applications in which a force generated during gait, walking, e.g. a ground reaction force, should be measured as accurately as possible to determine the gait from it. For this purpose, a sensor arrangement may be applied to a shoe in order to measure the generated force. Depending on a desired accuracy of the measurement, the sensor arrangement may include a number of sensor elements.

Within the shoe, the sensor elements may be, for example, embedded in a polymer foam. Embedding in foam may lead to mechanical stress to the sensor arrangement, because the foam causes e.g. a shear stress, tensile stress, or the like, which mechanically stresses a sensor film layer of the number of sensor elements and/or a wire connected to the number AO:TE sensor elements. Furthermore, the foam causes a rather non-linear sensor characteristic of the sensor arrangement, which needs to be taken into account during sensor signal processing, so that computationally intensive, functionally complex calibration and/or compensation may be required.

### SUMMARY OF THE INVENTION

There may, therefore, be a need for providing improved means to integrate a sensor arrangement in a shoe. An object of the invention is to provide a sensor arrangement applicable to a shoe, providing durability and/or accurate force measurement.

This object is solved by the subject-matter of the independent claims, wherein further embodiments are defined in the dependent claims.

In a first aspect, there is provided a sensor arrangement for detecting at least one force between a foot and a supporting surface. The sensor arrangement may be configured for mobile gait analysis, or the like. The sensor arrangement comprises a number of foil-based sensor elements, arranged as individual elements in an at least substantially common plane formed by a carrier. Further, the sensor arrangement comprises a number of pad elements, the number of which corresponds to the number of sensor elements, and which are arranged as individual elements in a manner that an area extension of a respective one of the number of pad elements at least partly overlaps with an area extension of a corresponding one of the number of sensor elements.

In this way, a pad element covers only one side of a respective one of the number of sensor elements instead of completely surrounding it, as opposed to embedding the sensor element in a foam. This may reduce mechanical stress on the sensor element, as the effect of tensile forces and/or shear forces is at least reduced, thereby increasing durability. Further, mechanical stress on wires connected to the sensor elements may be reduced, as the wires may be routed around the pad elements. Furthermore, the individually arranged pad elements allow a higher proportion of force to be detected than is possible when embedded in a foam, particularly when the foam simultaneously embeds several sensor elements, since the force dissipation and/or reaction is more direct than when embedded in a foam. As a result, the sensor arrangement of the first aspect provides an accurate force measurement. While the foam behaves nonlinearly due to its arrangement and/or material properties, the arrangement and/or material properties of the number of pad elements allow to reduce the effort regarding calibration and/or compensation of a nonlinear signal characteristic.

As used herein, the foil-based sensor element may utilize a force sensing resistor (FSR) technology, or any other kind of a layer arrangement of foils and conductive and/or semi conductive materials, and may be configured to generate a sensor signal indicative of a force generated when a foot strikes a floor surface.

For example, the sensor element may comprise: a first layer comprising an electrically non-conductive carrier foil and a first semi-conductive layer extending at least partially along a longitudinal direction of the first layer and being in contact with the electrically conductive area, a layer comprising a plurality of electrically conductive sections, at least one electrical connection area, and a second layer comprising an electrically non-conductive carrier foil, and a second semi-conductive layer extending at least partially along a longitudinal direction of the second layer, wherein at least one electrical connection area is in contact with the one semi-conductive layer of the first and second layer and being adapted to be connected to an electric energy source, and wherein the first semi-conductive layer and the second semi-conductive layer are arranged at a distance from one another and are adapted to selectively contact each other depending on the force applied to the sensor element, thereby changing a resistance value of the sensor element.

Further, each sensor element of the sensor arrangement may be configured to output an assigned signal that can change according to a load condition caused by the applied force. The carrier foil of the first and/or second layer may be formed from a suitable plastic, such as polyester or the like, and may have a thickness of about 50-150 microns (µm), preferably about 80-120 µm, preferably about 100 µm. The first and/or second semi-conductive layer may be formed as ink, in particular as carbon ink. The electrically conductive area may be formed by an electrically conductive ink, such as a silver-containing ink or silver ink, or the like. The electrically conductive area may serve as a connection to an electric circuit and may further serve as a conductor and connector of a carbon intermediate layer that may be arranged between the first and the second layer. This carbon layer may have a defined electrical resistance, which may be determined by the surface as well as by the mixture. The resistance of this surface may be chosen depending on the application. Silver has a higher electrical conductivity than graphite, for example, which significantly reduces the resistance, especially the input impedance. For further details and/or embodiments of the sensor element, reference is made to WO 2020/224937 A1, the content of which is incorporated herein in its entirety. A pad element may also be understood as a kind of stamp, configured to introduce the force into an adjacent part of a shoe, which may be the insole. Preferably, the pad element is only in contact with a flat side of the associated sensor element, wherein the flat side can be formed by a foil surface. The pad element may be, for example, plate-shaped, cuboid-shaped, etc., with a planar side having substantially the size or area of the corresponding planar side of the assigned sensor element. Individually arranged may be understood that the elements are not connected to each other at all, or at least only so weakly that hardly any force can be transmitted between the single pad elements.

As used herein, the overlapping of a respective one of the number of pad elements and the corresponding one, i.e. the assigned one, of the number of sensor elements, may be with respect to a force application direction. Preferably, surfaces of the pad elements and the sensor elements facing each other may be (essentially) the same and/or have (essentially) the same surface area.

Preferably, the number of sensor elements and/or sensor elements may be n, wherein n is an integer greater than 1 (one). In other words, the sensor arrangement may comprise multiple sensor elements. These may be arranged distributed over an imaginary foot surface to which the carrier is geometrically adapted to achieve an accurate measurement of the force(s) to be measured. The sensor arrangement may also be referred to as a sensor array.

Further, the individual pad element may be attached to the individual sensor element.

According to an embodiment, exactly one of the number of pad elements may be assigned to exactly one of the number of sensor elements. In other words, one single sensor element is attached with one single pad element. In this way, the pad elements are not connected to each other at all, and only interact with a single sensor element, so that particularly accurate force measurement is possible. In addition, a single pad element exerts hardly any mechanical stress on a single sensor element and/or the wires or wiring connecting the sensor elements.

In an embodiment, each of the number of sensor elements together with an assigned one of the number of pad elements may be arranged to be free-standing with respect to the other of the number of sensor elements and their assigned pad elements. In this way, the pad elements are not connected to each other at all, and only interact with a single sensor element. Further, in this way, the pad elements do not exert any mechanical stress onto the wires or wiring connecting the sensor elements.

According to an embodiment, the number of pad elements and the number of sensor elements may be attached to each other at least partially and/or in sections. This may be any type of locking suitable to fix a respective one of the number of pad elements and a corresponding one of the number of sensor elements to each other. For example, the attachment may be material-locking, e.g. by using an adhesive or the like, or form-fit, by, for example, the interaction of a projection and a recess, or the like. etc. This prevents the pad element from shifting or moving relative to the sensor element, and vice versa.

In an embodiment, a respective one of the number of pad elements may be applied on a side of the carrier facing the supporting surface. For example, if the side of the sensor arrangement or sensor elements facing the foot is a top side, then the pad element may be arranged on the bottom side thereof. Alternatively, if the side of the sensor arrangement or sensor elements facing the foot is a bottom side, then the pad element may be arranged on the top side thereof. This allows the force to be applied to the sensor element effectively.

According to an embodiment, a material of the number of pad elements may have at least elastic or rubber elastic properties. For example, the material may be a rubber, a silicon, or the like. This provides high durability. In addition, the influence of the pad element on the force measurement signal can be estimated.

In an embodiment, the material of the number of pad elements may have a shore A hardness in the range of 10 to 60, preferably in the range of 20 to 50, further preferably in the range of 25 to 45, yet further preferably in the range of 30 to 40, and most preferably of 35. This causes the material to be flexible, dense, and robust. This provides high durability. In addition, the influence of the pad element on the force measurement signal can be estimated.

According to an embodiment, the number of sensor elements and/or the number of pad elements may be between 2 and 30 or between 3 and 30, preferably between 13 and 18, and most preferably is 15 or 16. These may be arranged distributed over an imaginary foot surface to which the carrier is geometrically adapted to achieve an accurate measurement of the force(s) to be measured. By this, the sensor arrangement may provide information to implement a body scale, by calculating the total force detected through a number of sensor elements of the sensor arrangement. Further, the number of sensor elements and/or the number of pad elements may be arranged in the direction of the toe on several separate tracks running in the longitudinal direction of the foot, which can resemble fingers, in the midfoot area and in the heel area.

In an embodiment, the material thickness of the pad elements is between about 1 and 10 mm, preferably between about 1 and 8 mm, more preferably between about 2 and 5 mm, most preferably between 3 and 4 mm.

In an embodiment, the carrier may be formed by at least one foil. Wires may be embedded, molded or printed into the foil. Further, the foil may be multilayered.

According to an embodiment, the number of sensor elements may be surrounded by a border having a thickness, with respect to a direction of application of force to the sensor elements, that is smaller than or equal to a thickness of the number of pad elements. A material of the border may be the same as of the number of pad elements. In this way, buckling of a cover sole, which may be arranged on the sensor arrangement, e.g. in the direction of an insole, may at least be minimized.

According to a second aspect, there is provided a sole arrangement for a shoe. The sole arrangement comprises a sensor arrangement according to the first aspect and at least one sole element. Thereby, the sensor arrangement and the at least one sole element are arranged to float relative to each other.

By the floating arrangement, one or more foils of the sensor arrangement and/or sensor element have maximum possibilities for deflection under load, i.e. high sliding dynamics. In other words, the cover sole and/or the foot "floats" on the sensor arrangement. As a result, the foot/shoe movement does not cause significant tension in the one or more foils of the sensor arrangement and/or sensor element and/or the wires or wiring connecting the sensor elements . This provides high durability.

The shoe may be a sports shoe, running shoe, walking shoe, suit shoe, etc.

In an embodiment, the carrier of the sensor arrangement and the at least one sole element may be essentially spaced apart by the layer of formed by each pad element, wherein, preferably, the space between the carrier and the at least one sole element may be between about 1 and 10 mm, preferably between about 1 and 8 mm, more preferably between about 2 and 5 mm, most preferably between 3 and 4 mm.

According to an embodiment, the at least one sole element may comprise or may form a cover sole, also referred to as an upper sole, arranged floating to the sensor arrangement, and made of a material having a Shore A hardness higher than a material of a number of a pad elements of the sensor arrangement. The Shore A hardness may be at least 60, preferably between 90 and 100, most preferably 95. The foot is thus placed on the cover sole and the body weight, i.e. the sum of the force to be measured by each sensor element, is transferred exclusively via the number of sensor elements and the number of pad elements.

In an embodiment, the at least one sole element may comprise or may form an insole, also referred to as a lower sole, made of a material having a Shore A hardness higher than a material of a number of pad elements of the sensor arrangement. The Shore A hardness may be at least 60, preferably between 90 and 100, most preferably 95.

Preferably, the cover sole and/or the insole may have a surface extension corresponding to that of the sensor arrangement. The surface(s) may be slightly larger, but it should be avoided that a force component is transferred into side walls of the shoe by a too wide or long sole.

In an embodiment, the cover sole, as well as the lower sole may have a surface extension that is slightly larger than the surface extension of the sensor arrangement, preferably with the surface extension of the cover sole being slightly larger than the one of the lower sole.

According to an embodiment, at least a part of the number of pad elements may be connected to the insole by material-locking. Alternatively or additionally, at least a part of the number of pad elements may be molded into the insole. In this way, the number of pad elements and/or the sensor arrangement may be fixed on one side while the opposing side is floating with the cover sole. This provides high durability.

According to a third aspect, a detection system for detecting a force between a foot and a supporting surface is provided. The system comprises a shoe, a sole arrangement according to the second aspect, comprising a sensor arrangement according to the first aspect. Further, the system comprises a power supply, connected to the sensor arrangement, and an evaluation circuit, connected to the sensor arrangement. Thereby, the power supply and/or the evaluation circuit are accommodated in a material recess of the shoe and/or at least one sole element of the sole arrangement.

In this way, there is provided a system applied to a shoe, providing durability and/or accurate force measurement. Further, accommodating the power supply and/or evaluation circuit in the material recess facilitates fixing the sole arrangement to the shoe.

In an embodiment, the evaluation circuit is configured to operate as a body scale by calculating the total force detected through a number of sensor elements of the sensor arrangement. The sensor arrangement as explained above may provide accurate force measurement via each of the number of sensor elements. Summing up the individual sensor signals or value gives or translates to the total body weight force. For this purpose, the evaluation circuit may be configured to process the sensor signals of the number of sensor elements. Calculating a body weight may be performed by the evaluation circuit and/or an external evaluation software, or the like.

According to a fourth aspect, there is provided a method for providing a sensor arrangement. The sensor arrangement is configured to detect a force between a foot and a supporting surface. The method comprises the steps of:
arranging a number of foil-based sensor elements as individual elements in an at least substantially common plane formed by a carrier;
arranging a number of pad elements, the number of which corresponds to the number of sensor elements, as individual elements in a manner that an area extension of a respective one of the number of pad element at least partly overlaps with the area extension of a corresponding one of the number of sensor elements.

It is noted that the above embodiments may be combined with each other irrespective of the aspect involved. Accordingly, the method may be combined with structural features of the arrangement of the other aspects and, likewise, the arrangement of the first, second and third aspect may be combined with features of each other, and may also be combined with features described above with regard to the method according to the fourth aspect.

These and other aspects of the present invention will become apparent from and elucidated with reference to the embodiments described hereinafter.

### BRIEF DESCRIPTION OF THE DRAWINGS

Exemplary embodiments of the invention will be described in the following with reference to the drawings.
Fig 1 shows in a perspective top view a sensor arrangement according to an embodiment.
Fig. 2 shows in a perspective bottom view a sensor arrangement according to an embodiment.
Fig. 3 shows in a cross-section a sensor arrangement and a sole arrangement according to an embodiment.
Fig. 4 shows in a cross-section a sensor arrangement, a sole arrangement, and a detection system according to an embodiment.
Fig. 5 shows in a cross-section a sensor arrangement, a sole arrangement, and a detection system according to an embodiment.
Fig. 6 shows in a flow chart a method according to an embodiment.

### DETAILED DESCRIPTION OF EMBODIMENTS

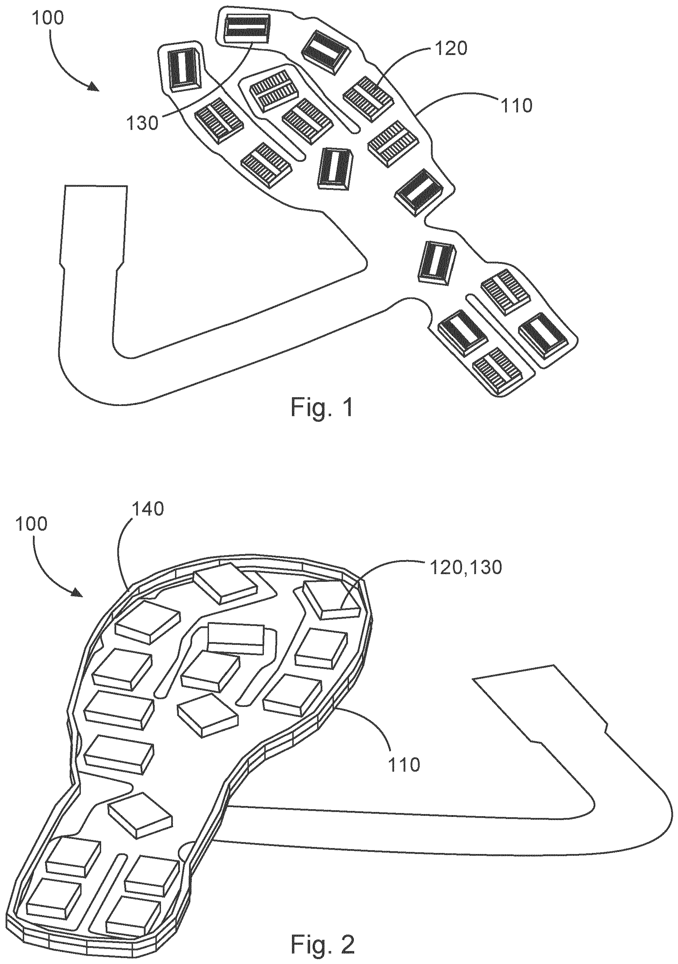

Fig. 1 shows in a perspective top view a sensor arrangement 100, which may also be referred to as a sensor array, configured to detect a force between a foot and a supporting surface, according to an exemplary embodiment. The force to be measured may also be referred to as ground reaction force.

The sensor arrangement 100 comprises a carrier 110. The carrier 110 may be a foil or laminate.

Further, the sensor arrangement 100 comprises a number of foil-based sensor elements 120, arranged as individual elements in an at least substantially common plane formed by the carrier 110. The number of sensor elements 120 may be a FSR sensor or another arrangement of foil, conductive, non-conductive and/or semi-conductive elements configured to measure a compressive force. The number of sensor elements 120 is between 2 and 30 or between 3 and 30, preferably between 13 and 18, and most preferably is 15 or 16. In the exemplary embodiment shown in Fig. 1, the number of sensor elements 120 is 16. According to Fig. 1, the sensor elements 120 are arranged distributed over the plane defined by the carrier 110.

The sensor arrangement 100 further comprises a number of pad elements 130. The number of pad elements 130 corresponds to the number of sensor elements 120. Accordingly, the number of pad elements 130 is between 2 and 30 or between 3 and 30, preferably between 13 and 18, and most preferably is 15 or 16. In the exemplary embodiment shown in Fig. 1, the number of pad elements 130 is 16. Further, the number of pad elements 130 are arranged as individual elements in a manner that an area extension of a respective one of the number of pad elements at least partly overlaps with an area extension of a corresponding one of the number of sensor elements. In other words, exactly one of the number of pad elements 130 is assigned to exactly one of the number of sensor elements 120. According to Fig. 1, each of the number of sensor elements 120 together with an assigned one of the number of pad elements 130 is arranged to be free-standing with respect to the other of the number of sensor elements 120 and their assigned pad elements 130. Therefore, around the individual pad elements 130 is a space for laying wires or wiring (not shown). A material of the number of pad elements 130 has at least elastic or rubber elastic properties. Preferably, the material of the number of pad elements may have a shore A hardness in the range of 10 to 60, preferably in the range of 20 to 50, further preferably in the range of 25 to 45, yet further preferably in the range of 30 to 40, and most preferably of 35.

According to Fig. 1, the number of sensor elements 120 and the number of pad elements 130 are arranged one above the other. Preferably, the surface areas of each one of the number of pad elements 130 and each one of the sensor elements 120 facing each other are congruent with each other or at least approximately the same size.

Preferably, the number of pad elements 130 and the number of sensor elements 120 are attached to each other at least partially and/or in sections. For example, there may be provided a suitable locking mechanism, such as material-locking, e.g. by using an adhesive, or a form lock.

According to Fig. 1, a respective one of the number of pad elements 130 is applied on a side of the carrier facing the supporting surface and/or the corresponding one of the number of sensor elements.

Fig. 2 shows in a perspective bottom view a further exemplary embodiment of the sensor arrangement 100. Accordingly, the number of sensor elements 120 and/or the number of pad elements 130 may be surrounded by a border 140 having a thickness, with respect to a direction of application of force to the sensor elements 120, that is smaller than or equal to a thickness of the number of pad elements 130. A material of the border 140 may be the same as of the number of pad elements 130, or may at least have similar material properties. Further, the material of the number of pad elements 130, having at least elastic or rubber elastic properties, has a shore A hardness in the range of 10 to 60, preferably in the range of 20 to 50, further preferably in the range of 25 to 45, yet further preferably in the range of 30 to 40, and most preferably of 35.

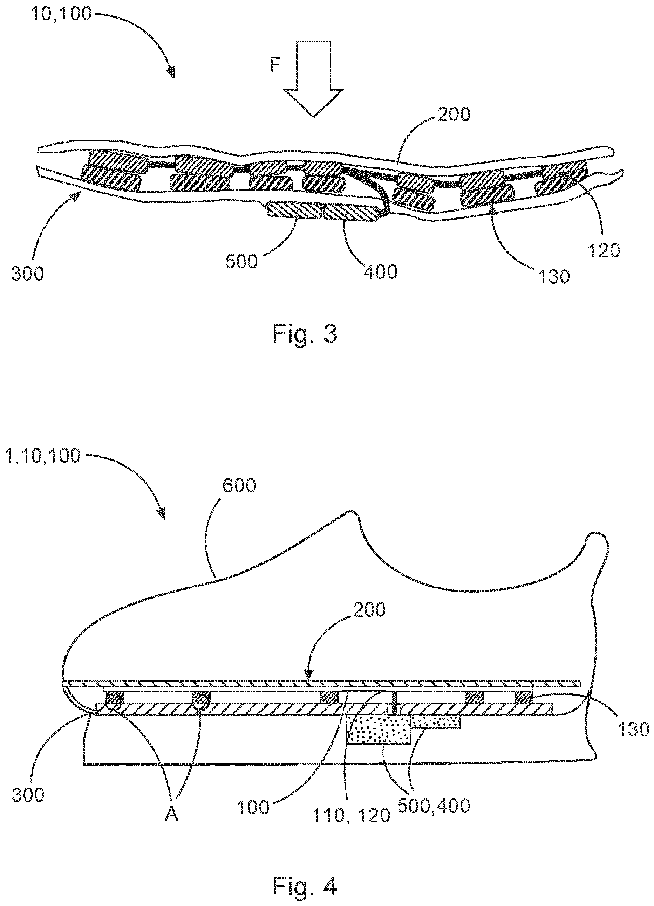

Fig. 3 shows in a cross-section the sensor arrangement 100 according to one or more of the above embodiments. Thereby, the sensor arrangement 100 is part of a sole arrangement 10. Accordingly, the sole arrangement 1, which is configured to be applied to a shoe, comprises the sensor arrangement 100 and at least one sole element 200, 300. In the exemplary embodiment of Fig. 3, a first sole element 200 comprises or forms a cover sole, i.e. an upper sole, arranged floating to the sensor arrangement 100. Further, the sole element 200 may be made of a material having a Shore A hardness higher than a material of a number of a pad elements 130 of the sensor arrangement 100. Further, a second sole element 300 comprises or forms an insole, i.e. a lower sole. It may be made of a material having a Shore A hardness higher than a material of the number of pad elements 130 of the sensor arrangement 100.

According to Fig. 3, a force F may be applied in the direction indicated by an arrow. In this direction, an area extension of a respective one of the number of pad elements 130 at least partly overlaps with an area extension of a corresponding one of the number of sensor elements 120. Further, the number of pad elements 130 and the number of sensor elements 120 are arranged one above the other. The first sole element 200, i.e. the cover sole, is arranged floating on, i.e. above, the sensor arrangement 100. The second sole element 300 carries and/or supports the number of pad elements 130, and is therefore arranged below the sensor arrangement 100.

Further, Fig. 3 shows a power supply 400, connected to the sensor arrangement 100; and an evaluation circuit 500, connected to the sensor arrangement 100. The evaluation circuit 500 is configured to evaluate the signals of the number of sensor elements 120.

Fig. 4 shows in a cross-section the sensor arrangement 100 according to one or more of the above embodiments. Thereby, the sensor arrangement 100 is part of a sole arrangement 10 and/or a detection system 1 for detecting a force between a foot and a supporting surface. The detection system 1 comprises a shoe 600, the sole arrangement 10 that comprises the sensor arrangement 100. Further, the detection system 1 comprises the power supply 400 and the evaluation circuit 500. According to Fig. 4, the power supply 400 and the evaluation circuit 500 are accommodated in a material recess of at least one sole element of the shoe 600 and/or the sole arrangement.

Further, according to Fig. 4, at least some or all of the pad elements 130 are attached to a corresponding one of the number of sensor elements 120, as designated in Fig. 4 by reference sign A. The attachment may be provided by a material-lock, by using e.g. an adhesive. Alternatively or additionally, the attachment may be provided by a form-lock, where the respective one of the pad elements 130 may have a protrusion and the corresponding one of the sensor elements 120 may have a material recess.

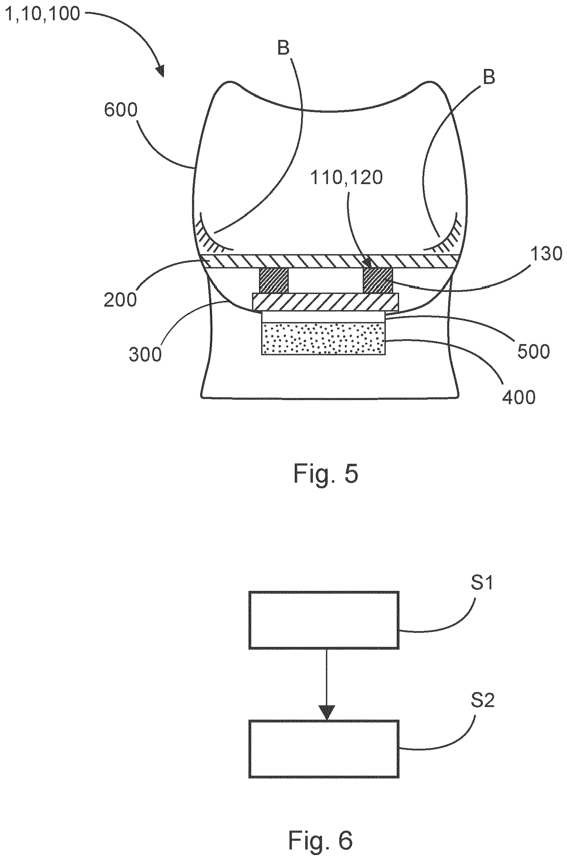

Fig. 5 shows in a cross-section the sensor arrangement 100 according to one or more of the above embodiments, and particularly the detection system 1 according to Fig. 4. According to Fig. 5, the first sole element 200, i.e. the cover sole, may be in close contact to the shoe 600, preferably to the side wall of the shoe 600 or may be attached and/or fixed to the shoe 600, preferably to the side wall of the shoe 600, by e.g. a material-locking, by e.g. using an adhesive. This attachment or fixation is designated in Fig. 5 by reference sign B. Being in close contact or being attached or fixed to the shoe 600, or its side wall, allows the sensor arrangement 100 or the sensor elements 120 to be protected from e.g. dirt. Further, the power supply 400 and or the evaluation circuit 500 are arranged within a material recess of the shoe 600.

From a functional perspective, the evaluation circuit 500 may be configured to operate as a body scale by calculating the total force detected through the number of sensor elements 120 of the sensor arrangement 100. The calculation may comprise summing up the individual forces of the number of sensor elements 120.

Fig. 6 shows in a flow chart a method for providing the sensor arrangement 100 according to any one of the above embodiments. The method comprises in a first step S1 arranging the number of foil-based sensor elements 120 as individual elements in an at least substantially common plane formed by the carrier 110. The method further comprises in a second step S2 arranging the number of pad elements 130, the number of which corresponds to the number of sensor elements 120, as individual elements in a manner that an area extension of a respective one of the number of pad elements 130 at least partly overlaps with the area extension of a corresponding one of the number of sensor elements 120.

It is noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

## Claims

1. A sensor arrangement (100) for detecting a force between a foot and a supporting surface, comprising:
a number of foil-based sensor elements (120), arranged as individual elements in an at least substantially common plane formed by a carrier (110);
a number of pad elements (130), the number of which corresponds to the number of sensor elements (120), and which are arranged as individual elements in a manner that an area extension of a respective one of the number of pad elements (130) at least partly overlaps with an area extension of a corresponding one of the number of sensor elements (120).

2. The sensor arrangement (100) of claim 1, wherein exactly one of the number of pad elements is assigned to exactly one of the number of sensor elements.

3. The sensor arrangement (100) of claim 1 or 2, wherein each of the number of sensor elements together with an assigned one of the number of pad elements is arranged to be free-standing with respect to the other of the number of sensor elements and their assigned pad elements.

4. The sensor arrangement (100) of any one of the preceding claims, wherein the number of pad elements and the number of sensor elements are attached to each other at least partially and/or in sections.

5. The sensor arrangement (100) of any one of the preceding claims, wherein a respective one of the number of pad elements (130) is applied on a side of the carrier (110) facing the supporting surface.

6. The sensor arrangement (100) of any one of the preceding claims, wherein a material of the number of pad elements (130) has at least elastic or rubber elastic properties.

7. The sensor arrangement (100) of any one of the preceding claims, wherein the material of the number of pad elements (130) has a shore A hardness in the range of 10 to 60, preferably in the range of 20 to 50, further preferably in the range of 25 to 45, yet further preferably in the range of 30 to 40, and most preferably of 35.

8. The sensor arrangement (100) of any one of the preceding claims, wherein the number of sensor elements (120) and/or the number of pad elements (130) is between 3 and 30, preferably between 13 and 18, and most preferably is 15 or 16.

9. The sensor arrangement (100) of any one of the preceding claims, wherein the carrier (110) is formed by at least one foil.

10. A sole arrangement (10) for a shoe, comprising,
a sensor arrangement (100) of any one of the preceding claims; and
at least one sole element (200, 300);
wherein the sensor arrangement (100) and the at least one sole element (200, 300) are arranged to float relative to each other.

11. The sole arrangement of claim 10, wherein the at least one sole element (200) comprises a cover sole, arranged floating to the sensor arrangement (100), and made of a material having a Shore A hardness higher than a material of a number of a pad elements (130) of the sensor arrangement (100).

12. The sole arrangement of claim 10 or 11, wherein the at least one sole element (300) comprises an insole, made of a material having a Shore A hardness higher than a material of a number of pad elements of the sensor arrangement.

13. A detection system (1) for detecting a force between a foot and a supporting surface, comprising:
a shoe (600);
a sole arrangement (10) of any one of claims 10 to 12, comprising a sensor arrangement (100) of any one of claims 1 to 9;
a power supply (400), connected to the sensor arrangement (100); and
an evaluation circuit (500), connected to the sensor arrangement (100),
wherein the power supply (400) and the evaluation circuit (600) are accommodated in a material recess of at least one sole element of the shoe (600) and/or the sole arrangement (10).

14. The detection system of claim 13, wherein the evaluation circuit (500) is configured to operate as a body scale by calculating the total force detected through a number of sensor elements (120) of the sensor arrangement (100).

15. A method for providing a sensor arrangement (100), the sensor arrangement (100) being configured to detect a force between a foot and a supporting surface, the method comprising:
arranging (SI) a number of foil-based sensor elements (120) as individual elements in an at least substantially common plane formed by a carrier (110);
arranging (S2) a number of pad elements (130), the number of which corresponds to the number of sensor elements (120), as individual elements in a manner that an area extension of a respective one of the number of pad elements (130) at least partly overlaps with the area extension of a corresponding one of the number of sensor elements (120).
